# FASCICULE DE BREVET EUROPEEN

(11) **EP 1 818 199 B1**
(45) Date de publication et mention de la délivrance du brevet: **13.03.2013**
(21) Numéro de dépôt: 07101739.6
(22) Date de dépôt: 05.02.2007
(51) Int. Cl.: B60H 3/00

(54) **Installation de ventilation, de chauffage et/ou de climatisation agencée pour la réception d'une cartouche de traitement de l'air par diffusion d'un agent traitant volatil**
Anlage zur Lüftung, Heizung und/oder Klimatisierung, die zur Aufnahme einer Patrone zur Luftaufbereitung durch Diffusion einer flüchtigen Aufbereitungssubstanz eingerichtet ist
Ventilation, heating and/or air-conditioning installation arranged to receive a cartridge for air treatment by distributing a volatile treating agent

(30) Priorité: 08.02.2006 FR 0601127
(43) Date de publication de la demande: 15.08.2007
(73) Titulaire: Valeo Systèmes Thermiques, 78321 Le Mesnil St Denis Cedex (FR)
(72) Inventeur: Giraud, Frédéric, 78610 Le Perray en Yvelines (FR); Ladrech, Frédéric, 78310 Maurepas (FR); Elliot, Gilles, 91080 Courcouronnes (FR)
(74) Mandataire: Léveillé, Christophe

(56) Documents cités:
- DE-A1- 10 346 182
- DE-U1- 20 118 329
- FR-A1- 2 833 531
- FR-A1- 2 868 017
- JP-A- 2004 210 087
- US-A1- 2002 074 421
- US-A1- 2003 186 643

## Description

### Domaine technique de l'invention.

L'invention est du domaine des installations de ventilation, de chauffage et/ou de climatisation, pour habitacle de véhicule notamment. Elle a pour objet une telle installation, qui est agencée pour recevoir un dispositif de traitement de l'air qui circule à son travers, par diffusion d'un agent traitant volatil à l'encontre d'un développement de microorganismes.

### Etat de la technique.

On rappelle qu'une installation de ventilation, de chauffage et/ou de climatisation pour véhicule automobile comporte un circuit d'air entre au moins une bouche d'admission et au moins une bouche d'évacuation, sur lequel circuit est interposé divers moyens de traitement de l'air. Cette bouche d'admission d'air est une entrée d'air extérieur et/ou une entrée d'air recyclé. Les moyens sont notamment des moyens pulseurs pour mettre en mouvement un flux d'air à travers le circuit, des moyens de chauffage et/ou de refroidissement et des moyens d'épuration de l'air, tels que par filtration à particules, par ionisation, par photocatalyse ou analogue. Parmi ces moyens de traitement, on connaît ceux mettant en oeuvre un agent traitant volatil. De manière courante, l'agent traitant est un produit sous forme liquide ou gélifié supporté par un substrat et destinés à éviter le développement de microorganismes pour lutter contre les mauvaises odeurs qui sont une source d'inconfort pour les passagers. De tels microorganismes se développent plus particulièrement dans la zone d'un évaporateur que comporte l'installation. Cet agent traitant a donc des propriétés anti-septiques.

Se pose le problème de l'implantation dans le circuit des moyens de traitement de l'air véhiculé par diffusion d'un agent volatil.

Une telle implantation ne doit pas être faite au détriment de l'encombrement de l'installation souhaité le plus restreint possible ni être source d'un surcoût conséquent. Toujours au regard des coûts, il est souhaité d'organiser cette implantation de manière à permettre une maintenance aisée des moyens de traitement, notamment en facilitant leur accès pour le remplacement du substrat porteur de l'agent traitant, voire de l'agent traitant lui-même lorsque ce dernier est épuisé. Toujours au regard des coûts, cette implantation ne doit pas être réalisée à l'encontre d'une standardisation de l'installation qu'il est souhaitable d'organiser de manière à pouvoir être intégrée sans modification majeure sur de quelconques véhicules et plus particulièrement tant sur des véhicules d'une quelconque plateforme d'un constructeur que sur des véhicules de constructeurs différents afin de rentabiliser au mieux les efforts de conception de l'installation.

Par ailleurs, la diffusion de l'agent traitant dans le flux doit être régulière et fiable, en quantité adaptée pour procurer un traitement satisfaisant et pérenne.

DE 201 18 329 U1 divulgue une installation selon le préambule de la revendication 1.

Il a été proposé par DE10346182 (BEHR Gmbh & Co Kg) d'agencer les moyens de traitement en une réserve de l'agent traitant placée à l'intérieur du circuit d'air. Le renouvellement de l'agent traitant s'effectue par l'intermédiaire d'un canal reliant la réserve à l'extérieur du circuit. De telles dispositions sont délicates à mettre en oeuvre et ne sont pas satisfaisantes au regard des pertes de charge aéraulique ni au regard de la standardisation de l'installation.

Il a aussi été proposé par JP2004210087 (ZEXEL VALEO CLIMATE CONTROL CORP.) d'agencer les moyens de traitement en une cartouche fixée sur une paroi du circuit d'air. Cette cartouche est formée d'un support qui comporte un logement de réception du substrat qui est munie de moyens de fixation à la face extérieure d'une paroi délimitant le circuit. Cette dernière comporte dans son épaisseur une cavité débouchante ou non sur le volume intérieur du circuit d'air pour la réception de la partie de la cartouche comportant le logement de réception du substrat. Cependant, les modalités de diffusion de l'agent traitant méritent d'être améliorées pour procurer un traitement régulier, fiable et pérenne de l'air véhiculé par le circuit. En d'autres termes, la disposition de la cartouche dans le JP2004210087 ne permet pas d'offrir une surface d'échange satisfaisante. Il est rappeler à cet égard que la cartouche d'agent traitant est en fonctionnement quand le pulseur est arrêté, c'est-à-dire quand il n'y a pas ou peu de mouvement d'air dans l'installation. C'est pourquoi, la surface d'échange avec ce volume d'air immobile revêt une importance majeure. Par ailleurs, on notera que la place disponible sur un boîtier de climatisation est limitée de sorte que l'installation d'une cartouche d'agent traitant disposée dans le même plan que la paroi de l'installation est pratiquement impossible si l'on souhaite maintenir une diffusion satisfaisante sur une langue durée, par exemple un an.

### Objet de l'invention.

Le but de la présente invention est de proposer une installation de ventilation, de chauffage et/ou de climatisation, pour véhicule notamment, agencée pour recevoir un dispositif de traitement de l'air circulant à son travers par diffusion d'un agent traitant volatil. Il est plus particulièrement visé par la présente invention de proposer une telle installation dont les modalités d'implantation des moyens de traitement de l'air par diffusion d'un agent volatil permettent un renouvellement aisé et peu coûteux des moyens de traitement après épuisement de l'agent volatil, permettent une diffusion régulière en quantité satisfaisante de celui-ci et permettent une telle implantation en un quelconque endroit du circuit d'air de l'installation sans porter atteinte ni à la qualité du traitement obtenu ni à la standardisation de l'installation et sans induire un surcoût conséquent.

L'installation de la présente invention est une installation de ventilation, de chauffage et/ou de climatisation. Cette dernière comprend un circuit d'air entre au moins une bouche d'entrée et au moins une bouche de sortie. Ce circuit d'air est délimité par une capacité étanche à l'air de l'environnement extérieur, cette capacité étant ceinte par une paroi. Cette paroi est susceptible d'être une paroi d'un conduit ou d'un boîtier logeant des moyens de traitement de l'air, traitement thermique notamment. L'installation est équipée de moyens de traitement de l'air par diffusion d'un agent volatil qui sont placés sur le circuit d'air pour épurer l'air et lutter contre le développement de microorganismes notamment. Plus particulièrement, l'installation est du genre dans laquelle la paroi délimitant le circuit d'air comporte au moins une ouverture à son travers pour le passage d'une cartouche de traitement de l'air par émission d'un agent traitant volatil. Cette cartouche est du type comprenant un support d'un substrat imprégné de l'agent volatil. L'installation comprend une cloison perméable délimitant une chambre de diffusion de l'agent volatil qui est disposée dans le volume intérieur de la capacité du circuit d'air. La disposition dans le volume intérieur s'entend en ce que la cloison perméable dépasse de l'épaisseur de la paroi de l'installation pour déboucher dans le volume intérieur de cette installation. On comprend donc que cette paroi comprend une surface émettrice ou cloison en contact avec l'air sur au moins deux plans qui s'entrecoupent. Ces dispositions sont telles que l'agent traitant est susceptible d'occuper le volume intérieur de la chambre située dans le circuit d'air délimité par la paroi et de diffuser à travers la cloison dans le circuit d'air. La surface globale de la cloison offre à la fois une faculté de diffusion en quantité satisfaisante de l'agent traitant à l'intérieur du circuit tout en régulant cette diffusion selon une quantité quotidienne souhaitée.

Selon la présente invention, une telle installation est principalement reconnaissable en ce que ladite cloison est formée d'une matière plastique chargée de talc en proportion comprise entre 0% et 40% pour une épaisseur de cloison comprise entre 0.5 mm et 2 mm, ladite cloison étant ménagée au moins en partie sur la paroi du circuit d'air et ladite cloison est indifféremment rapportée et/ou intégrée de moulage à la paroi du circuit d'air pour former une pièce unitaire. On comprend donc que cette cloison est perméable afin d'assurer une diffusion déterminée de l'agent traitant.

Un espace de communication directe est notamment ménagé entre le substrat et la cloison pour la diffusion de l'agent traitant à l'intérieur de la chambre. Cet espace à l'intérieur duquel se répand l'agent volatil préalablement à sa diffusion à travers la cloison perméable est susceptible d'être formée par une zone périphérique de la chambre de diffusion qui est ménagée autour du substrat logé au moins en partie à l'intérieur de cette chambre de diffusion. Cet espace peut aussi être formé par une partie au moins de la chambre de diffusion, partie distale notamment, sinon par la totalité du volume de la chambre de diffusion dans le cas où le substrat est disposé hors de ce volume.

Plus particulièrement, la cloison est de préférence interposée entre le substrat et le volume de circulation de l'air à travers le circuit, la chambre de diffusion formant logement de réception du substrat.

Selon une variante de réalisation dans laquelle l'ouverture est ménagée à travers la paroi suivant un axe transversal au sens de circulation d'air à l'intérieur du circuit, la cloison s'étend plus particulièrement en prolongement et au voisinage de la périphérie de l'ouverture que comporte la paroi pour le passage de la cartouche On comprend donc que la cartouche s'étend sensiblement perpendiculairement à la direction de circulation du flux d'air.

Selon diverses variantes de réalisation, la cloison est ménagée au moins en partie sur la cartouche sinon complètement ou la cloison est ménagée au moins en partie sur la paroi du circuit d'air sinon complètement. La cloison est susceptible d'être formée à partir de cloisons élémentaires ménagées respectivement sur la cartouche et sur la paroi. La cloison, ou les cloisons élémentaires, sont selon divers exemples de réalisation indifféremment rapportées et/ou intégrées de moulage à la paroi du circuit d'air ou au support de la cartouche, selon les cas visés précédemment, pour former une pièce unitaire avec ces derniers. Selon une variante de réalisation, la cloison perméable est celle d'un réceptacle amovible de logement du substrat, qui est rapporté sur le support par l'intermédiaire de moyens de réception réversible, tel que par emboîtement, par clipage, par agrafage ou par la mise en oeuvre d'organes d'assemblage coopérants, tel que par vissage. Par exemple, le support comporte un berceau ou analogue de réception du réceptacle par emboîtement.

Selon différents cas d'implantation de la cartouche, celle-ci est par exemple installée sur la paroi d'un boîtier logeant au moins des moyens de traitement thermique de l'air ou encore est installée sur la paroi d'un conduit situé en amont d'un tel boîtier ou encore est installée sur la paroi d'un conduit situé en aval d'un tel boîtier. Le sens des termes amont et aval s'entend par rapport au sens de circulation du flux d'air à l'intérieur de l'installation.

Bien entendu, plusieurs cartouches sont susceptibles d'être installées en de quelconques endroits respectifs du circuit d'air et plus particulièrement sur la paroi d'un quelconque conduit ou du boîtier. En effet et sans sortir du cadre de l'invention, la cartouche peut être installée dans le boîtier d'entrée d'air de l'installation, dans ou au abord du pulseur ou dans le conduit d'amené d'air vers le boîtier contenant les échangeurs thermiques.

De même, la cartouche est susceptible d'être installée sur un circuit annexe d'apport d'air.

Selon diverses variantes de réalisation, la cartouche est une cartouche amovible interchangeable munie de moyens de fixation réversible avec la paroi ou encore est une cartouche équipée de moyens de jonction réversible entre le support et le substrat, ce dernier étant interchangeable ou encore est munie de moyens de rechargement du substrat en agent volatil. Il en ressort que le renouvellement en agent traitant est susceptible d'être opéré à partir d'un remplacement de la cartouche et/ou du substrat et/ou à partir d'un rechargement du substrat en agent traitant.

Plus particulièrement, les moyens de fixation de la cartouche sur la paroi sont susceptibles d'être des moyens de fixation irréversibles, tels que par collage, par soudage, par intégration de moulage du support à la paroi ou technique analogue par exemple. Selon une autre variante les moyens de fixation de la cartouche à la paroi sont susceptibles d'être des moyens de fixation réversibles tels que par emboîtement, par clipage, par vissage, par l'intermédiaire d'organes d'assemblages coopérants ou techniques analogues par exemple.

Les moyens de jonction entre le support et le substrat sont susceptibles d'être des moyens de jonction irréversibles tels que par collage ou technique analogue. Selon une autre variante, les moyens de jonction entre le support et le substrat sont susceptibles d'être des moyens de jonction réversibles, tels que par emboîtement, par clipage, par l'intermédiaire d'organes d'assemblages coopérants ou technique analogue par exemple. Selon encore une autre variante, les moyens de jonction entre le support et le substrat sont formés dudit réceptacle de réception du substrat, qui est rapporté sur le support, la paroi de ce réceptacle constituant ladite cloison perméable.

De préférence, le substrat est un corps cohésif spongieux. Ces dispositions permettent une implantation de la cartouche sur la paroi suivant une quelconque orientation, sans affecter la tenue naturelle du substrat.

On notera que l'agent traitant est susceptible d'être en outre un agent odorant.

### Description des figures.

La présente invention sera mieux comprise à la lecture de la description qui va en être faite d'exemples de réalisation, en relation avec les figures des planches annexées, dans lesquelles :
- la fig.1 est un schéma illustrant un premier exemple de réalisation de moyens de traitement équipant une installation de ventilation, de chauffage et/ou de climatisation, pour le traitement de l'air véhiculé à travers cette installation par un agent volatil,
- la fig.2 est un schéma illustrant un deuxième exemple de réalisation de moyens de traitement analogues à ceux représentés sur la fig.1,
- les fig.3 et fig.4 sont des schémas illustrant un troisième exemple de réalisation de moyens de traitement analogues à ceux représentés sur les fig.1 et fig.2, respectivement en coupe longitudinale et en coupe transversale,
- la fig.5 est un schéma illustrant un quatrième exemple de réalisation de moyens de traitement analogues à ceux représentés sur les figures précédentes,
- les fig.6 à fig.10 sont des illustrations de différents exemples d'implantation sur une paroi de l'installation d'une cartouche que comprennent les moyens de traitement représentés sur les figures précédentes, représentés de bout et en coupe longitudinale pour chacune de ces figures,
- la fig.11 est un autre exemple d'implantation d'une cartouche sur une paroi de l'installation, représenté en coupe longitudinale,
- la fig.12 est un schéma illustrant différents lieux d'implantation des moyens de traitement de l'invention sur une paroi d'un circuit d'air d'une installation de ventilation, de chauffage et/ou de climatisation d'un véhicule automobile

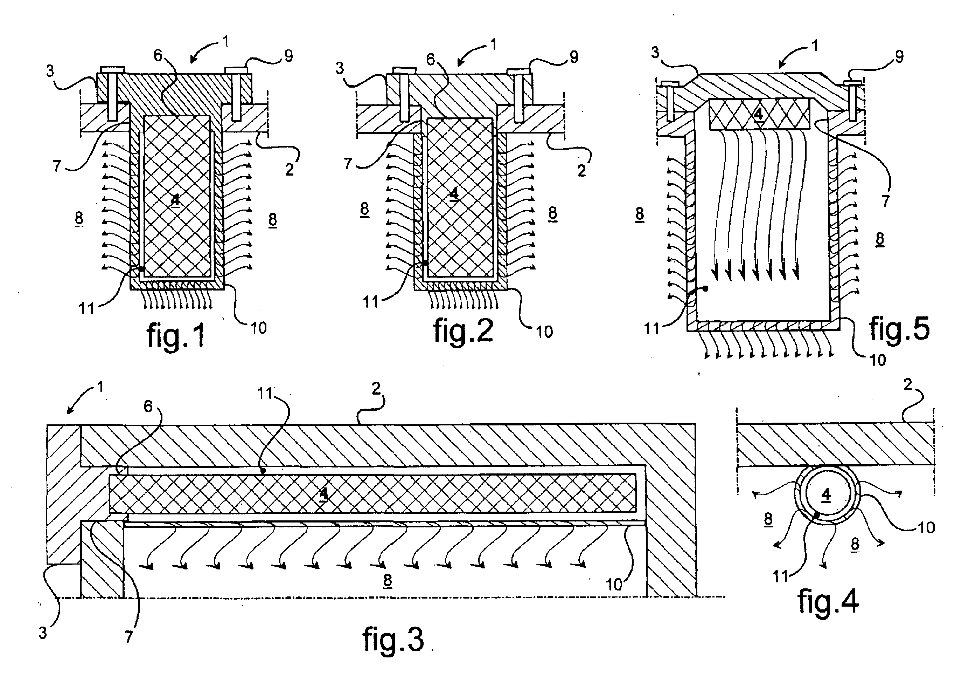

Sur les fig.1 à fig.4, des moyens sont destinés à équiper une installation de ventilation, de chauffage et/ou de climatisation d'un véhicule automobile pour le traitement de l'air véhiculé à travers cette installation pour lutter contre le développement de microorganismes. De tels moyens mettent en oeuvre un agent traitant volatil qui diffuse une substance active et anti-septique à l'intérieur du volume délimité par le circuit d'air de l'installation. Cet agent traitant peut accessoirement être mêlé à une substance odorante. Il est aussi envisageable de remplacer l'agent traitant par un produit odorant ou analogue pour limiter l'action des moyens de traitement à une fonction d'odorisation de l'air véhiculé par l'installation.

Ces moyens de traitement comprennent une cartouche 1 destinée à être installée sur une paroi 2 de l'installation. Cette cartouche 1 comprend un support 3 d'un substrat 4 imprégné de l'agent traitant volatil. Le substrat 4 est par exemple constitué d'un sachet perméable ou analogue contenant l'agent traitant à l'état liquide ou de gel. De préférence cependant, le substrat 4 est un corps cohésif spongieux autrement appelé mèche, avantageusement constitué d'une masse susceptible d'être formée à partir de matière textile, telles que fibres textiles mêlées naturelles ou artificielles, à partir d'une masse de cellulose, tel qu'un tampon de buvard épais du type formé d'une touche ou d'une mouillette, à partir d'un corps formé d'un substrat minéral ou végétal, éventuellement parcellisé et compressé, à partir d'une masse cohésive de poudre de polymère, voire à partir d'un matériau d'origine animale tel qu'un cuir poreux. Une telle organisation du substrat 4 en corps cohésif lui confère une tenue naturelle qui permet sa mise en place sur l'installation suivant une quelconque orientation, notamment au regard de la gravité. L'agent traitant est notamment de l'allyl-isothiocyanate ou produit analogue, à l'état liquide ou gélifié.

Le substrat 4 est rapporté sur le support 3 de manière amovible ou de manière irréversible. Par exemple, le substrat 4 est susceptible d'être emboîté à l'intérieur d'un logement 6 que comporte le support 3 en étant de préférence mais accessoirement collé à l'intérieur de ce logement 6 tel que représenté sur les fig.1 à fig.4. Sur la fig.5, le substrat 4 est fixé, tel que par collage ou analogue, sur une face du support 3 qui est orientée vers la paroi 2 de l'installation.

La paroi 2 de l'installation comporte une ouverture 7 à son travers pour le passage du substrat 4 porté par le support 3. Cette ouverture 7 est débouchante à la fois vers l'extérieur du circuit d'air 8 de l'installation dont la capacité est délimitée par la paroi 2 et vers l'intérieur du circuit d'air 8. Ces dispositions visent à permettre une installation aisée de la cartouche 1 sur la paroi 2, tout en laissant la possibilité à l'agent traitant de diffuser largement vers l'intérieur du circuit d'air 8 quand le flux d'air n'est pas mis en mouvement.

La cartouche 1 est fixée sur la paroi 2 par l'intermédiaire de moyens de fixation, tel qu'au moyen de vis 9 ou par emboîtement sur les exemples de réalisation illustrés sur les fig.1 à fig.5. De telles dispositions permettent non seulement une implantation aisée de la cartouche 1 sur l'installation mais aussi de pouvoir renouveler facilement l'agent traitant après épuisement, soit par remplacement de la cartouche 1, soit par remplacement du substrat 4, soit par rechargement du substrat 4 en agent traitant.

Pour réguler en quantité suffisante la diffusion de l'agent traitant à l'intérieur du circuit d'air 8 sans pour autant accroître la dimension de l'ouverture 7 ménagée à travers la paroi 2, une cloison perméable 10 est interposée entre le substrat 4 et le volume intérieur de la capacité délimitant le circuit d'air 8.

On notera que sur la figure 1, la cloison 10 ou surface émettrice fait partie intégrante du support 3 et sort d'un moulage commun. Le support 3 et la cloison 10 forme un ensemble unitaire. Par contre, la figure 2 montre une cloison 10 amovible qui peut être soit fixée sur la face intérieure de la paroi 2, soit sur la paroi périphérique du réceptacle 6.

Les figures 3 à 5 mettent en avant une différence avec les figures 1 et 2. En effet, la cloison émettrice 10 fait partie de la paroi 2, soit par formation d'une cavité en forme de « U » ouverte vers l'environnement extérieur, soit par formation d'une cavité sensiblement tubulaire sur la face interne de la paroi 2 de l'installation.

Cette cloison 10 délimite une chambre 11 de diffusion de l'agent traitant, qui est localisée à l'intérieur du circuit d'air 8. Il en ressort une diffusion de l'agent traitant à l'intérieur du circuit d'air 8 à travers une surface de diffusion largement supérieure à celle offerte par la seule surface de l'ouverture 7. En outre, il est rendu aisé d'ajuster cette diffusion à partir d'une modification des caractéristiques intrinsèques de la cloison perméable 10, tel que son épaisseur, ses dimensions et sa surface en relevant, ou son matériau constitutif. Selon l'installation de la présente invention, la cloison perméable 10 est formée d'une matière plastique, telle que par exemple du type polypropylène, chargée de talc en proportion comprise entre 0% et 40%, et plus particulièrement de l'ordre de 20%, pour une épaisseur de cloison comprise entre 0,5 mm et 2 mm. A titre indicatif et pour exemple, la cloison perméable 10 présente une épaisseur de l'ordre de 0,9 mm plus ou moins 20%, pour une surface globale de 4200 mm2 plus ou moins 10%. Pour un tel agencement de la chambre 11 délimitée par la cloison perméable 10, la diffusion annuelle de l'agent traitant est de l'ordre de 45 mg/jour à température constante de 40°C. On comprendra que ces indications de dimensions et de matériau constitutif de la cloison perméable 10, et de diffusion de l'agent traitant, sont susceptibles d'être modifiées sans sortir du cadre de l'invention.

Sur les fig.1 à fig.4, le substrat 4 se prolonge au-delà du support 3 pour être disposé dans le volume délimité par la paroi 2. Dans ce cas, le substrat 4 est logé à l'intérieur de la chambre de diffusion 11. De préférence, un espace est ménagé autour du substrat 4 entre celui-ci et la cloison perméable 10 pour permettre une diffusion de l'agent traitant à l'intérieur de la chambre 11 préalablement à sa diffusion à travers la cloison perméable 10 vers l'intérieur du circuit d'air. Sur la fig.5, le substrat 4 occupe un volume qui n'occupe pas le volume intérieur de la capacité de circulation d'air délimitée par la paroi 2. L'agent traitant diffuse à l'intérieur de la chambre 11 délimitée par la cloison perméable 10, préalablement à sa diffusion à travers cette dernière vers l'intérieur du circuit d'air 8.

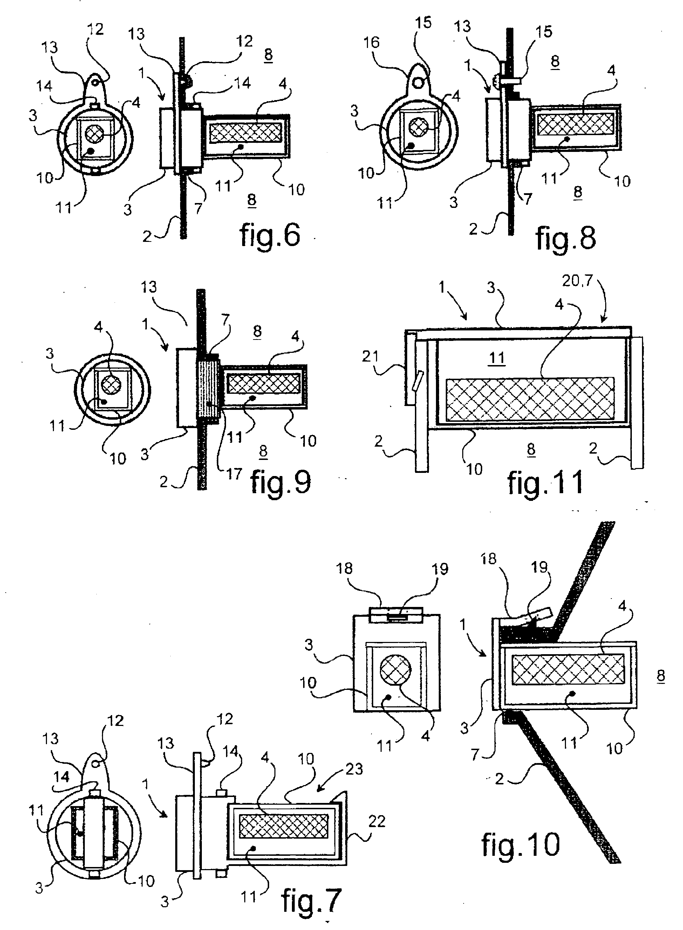

Sur les fig.6 à fig.10, le substrat 4 est rapporté sur le support 3 par l'intermédiaire d'un réceptacle contenant le substrat 4. La paroi de ce réceptacle constitue la cloison perméable 10.

Selon une première variante illustrée sur les fig.6 et fig.8 à fig.10, ce réceptacle est rapporté sur le support 3 de manière irréversible, par collage.

Selon la variante illustrée sur la fig.7, le support comporte un berceau 22 de réception d'un réceptacle 23 contenant le substrat 4. Ce berceau peut être ajouré de sorte à libérer une grande partie de la paroi du réceptacle 23 pour émettre largement dans le circuit 8.

Sur les fig.6 et fig.7, les moyens de fixation de la cartouche 1 sur la paroi 2 comprennent un picot d'emboîtement 12 ménagé sur une languette 13 que comporte la cartouche 1. Un dispositif 14 à ergots de liaison par quart de tour complète avantageusement la fixation de la cartouche 1 sur la paroi 2.

Sur la fig.8, les moyens de fixation de la cartouche 1 sur la paroi 2 sont du type par vissage, et comprennent une vis 15 qui traverse une languette 16 que comporte la cartouche 1 et qui coopère avec un trou taraudé que comporte la paroi 2.

Sur la fig.9, les moyens de fixation de la cartouche 1 sur la paroi 2 sont du type par vissage, et comprennent un filetage 17 ménagé sur le support 3 qui coopère avec un taraudage complémentaire ménagé dans l'ouverture 7.

Sur la fig.10, les moyens de fixation de la cartouche 1 sur la paroi 2 sont du type par clipage, et associent une languette 18 pourvue d'au moins un passage pour au moins un ergot 19. La languette 18 et l'ergot 19 sont indifféremment ménagés respectivement sur la cartouche 1 et/ou sur la paroi 2. Sur l'exemple de réalisation illustré, la cartouche 1 est munie de la languette 18 et coopère avec un ergot 19 ménagé sur la paroi 2.

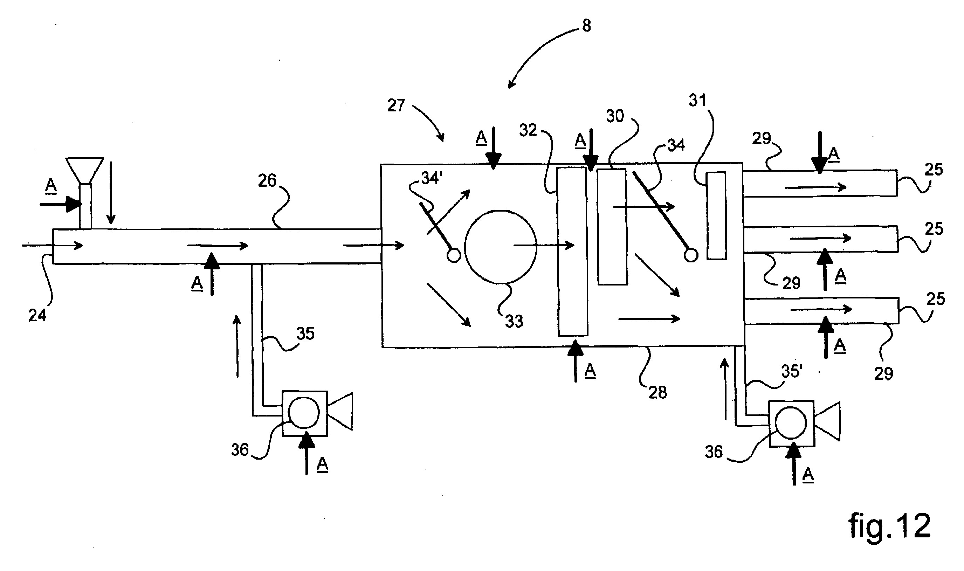

Sur la fig.11, la cartouche 1 est installée au débouché 20 d'un conduit que comporte le circuit d'air (visible sur figure 12 avec un conduit référencé 35 ou 35'). Dans ce cas, on comprendra que l'ouverture 7 que comporte la paroi 2 est formée par ledit débouché 20. Selon diverses variantes de réalisation, les moyens de fixation de la cartouche 1 sur la paroi 2 sont du type par clipage, du type par agrafage ou par pincement élastique de la paroi 2 entre deux organes d'appui antagonistes que comporte la cartouche 1. Sur l'exemple de réalisation illustré, les moyens de fixation comprennent une agrafe 21 de maintien de la cartouche 1 sur la paroi 2 bordant le débouché 20 du conduit.

Sur la fig.12, une installation de ventilation, de chauffage et/ou de climatisation, d'un véhicule notamment, comprend le circuit d'air 8 entre au moins une bouche d'entrée 24 à travers laquelle est admis de l'air. Cette bouche d'entrée est l'entrée d'air extérieure ou l'entrée d'air recyclé. Cette installation comprend une pluralité de bouches de sortie 25 destinées à l'aération de zones spécifiques de l'habitacle, voire d'un siège ou d'un autre organe du véhicule. Sur l'exemple schématique de réalisation illustré, le circuit d'air 8 comprend notamment un conduit 26 d'amenée de l'air vers un module 27 de traitement thermique, qui comprend un boîtier 28 logeant des moyens de traitement thermique et/ou d'épuration de l'air, et une pluralité de conduits 29 d'évacuation de l'air traité hors du boîtier 28 vers les zones et/ou organes à aérer.

Le boîtier 28 loge par exemple des organes caloporteurs pour le traitement thermique de l'air, tel qu'un évaporateur 30 et au moins un radiateur 31. Le boîtier 28 loge aussi au moins un filtre 32, à particules ou analogue, pour l'épuration de l'air. Ce filtre 32 est disposé en aval d'un pulseur 33 générant un flux d'air dans le circuit 8. Des volets 34, 34' de répartition du flux d'air à l'intérieur du boîtier 28 permettent de diriger l'air vers au moins l'un ou l'autre des organes caloporteurs 30, 31. Le circuit d'air, et notamment le boîtier 28, est aussi susceptible de loger d'autres organes de traitement de l'air sans sortir du cadre de l'invention, tel qu'un ionisateur, un dispositif de traitement par photocatalyse, un dispositif de traitement par précipitation électrostatique et/ou tout autre dispositif de traitement de l'air. L'organisation et la disposition des organes logés dans le boîtier 28 sont illustrées pour exemple, et peuvent être modifiées sans sortir du cadre de l'invention.

La cartouche peut être aisément implantée sur le circuit d'air en un quelconque endroit de celui-ci, repéré par les différentes flèches A. La localisation de cette implantation est susceptible d'être située en amont du module 27 de traitement thermique, dans la zone du conduit d'amenée 26 ou dans le boîtier 28, de préférence en amont de l'évaporateur 30 ou encore dans les conduits d'évacuation 29. On notera que les lieux d'implantation préférés de la cartouche sont situés dans les zones de l'installation facilement accessibles, pour permettre une maintenance aisée des moyens de traitement de l'air par diffusion de l'agent volatil et notamment en vue du renouvellement de ces moyens en agent volatil lorsque celui-ci est épuisé. Plus particulièrement, ces zones sont situées en amont de l'évaporateur 30, dans la zone d'entrée d'air et dans la zone du filtre à particules 32 ou dispositif d'épuration d'air analogue. L'implantation de la cartouche est aussi susceptible d'être située en aval d'un volet de recyclage 34' situé en amont du pulseur 33.

L'installation est aussi susceptible de comporter des circuits annexes 35, 35' d'apport d'air secondaire, tel qu'une prise d'air dans le compartiment moteur ou une prise d'air dans l'habitacle, située en amont et/ou en aval du module de traitement thermique 27. Ces circuits annexes 35, 35' sont des conduits à l'extrémité desquels est installé une cartouche d'agent traitant.

## Revendications

1. Installation de ventilation, de chauffage et/ou de climatisation comprenant un circuit d'air (8) entre au moins une bouche d'entrée et au moins une bouche de sortie, ce circuit d'air (8) étant délimité par une capacité étanche à l'air ceinte par une paroi (2) comportant au moins une ouverture (7) à son travers pour le passage d'une cartouche (1) de traitement de l'air par diffusion d'un agent traitant volatil, une cloison perméable (10) délimitant une chambre (11) de diffusion de l'agent volatil étant disposée dans le volume intérieur de la capacité du circuit d'air (8), l'agent traitant se diffuse à travers la surface globale de la cloison (10), **caractérisée en ce que** ladite cloison étant formée d'une matière plastique chargée de talc en proportion comprise entre 0% et 40% pour une épaisseur de cloison comprise entre 0.5 mm et 2 mm, ladite cloison (10) étant ménagée au moins en partie sur la paroi du circuit d'air (8) et ladite cloison (10) est indifféremment rapportée et/ou intégrée de moulage à la paroi (2) du circuit d'air (8) pour former une pièce unitaire.

2. Installation selon la revendication 1, **caractérisée en ce que** l'ouverture (7) étant ménagée à travers la paroi (2) suivant un axe transversal au sens de circulation d'air à l'intérieur du circuit (8), la cloison (10) s'étend dans le prolongement de l'ouverture (7) que comporte la paroi (2).

3. Installation selon la revendication 1, **caractérisée en ce que** l'ouverture (7) étant ménagée à travers la paroi (2) suivant un axe correspondant au sens de circulation d'air à l'intérieur du circuit (8), la cloison (10) s'étend transversalement à l'axe de l'ouverture (7).

4. Installation selon l'une quelconque des revendications précédentes, **caractérisée en ce que** la cloison (10) est interposée entre un substrat (4) de la cartouche (1) et le volume de circulation de l'air à travers le circuit (8), la chambre de diffusion (11) formant logement de réception du substrat (4).

5. Installation selon l'une quelconque des revendications précédentes, **caractérisée en ce que** la cartouche (1) est installée sur la paroi d'un boîtier (28) logeant au moins des moyens (30, 31) de traitement thermique de l'air.

6. Installation selon l'une quelconque des revendications précédentes, **caractérisée en ce que** la cartouche (1) est installée sur la paroi d'un conduit (26, 35) indifféremment situé en amont et/ou en aval d'un boîtier (28) logeant au moins des moyens (30, 31) de traitement thermique de l'air.

7. Installation selon l'une quelconque des revendications précédentes, **caractérisée en ce que** la cartouche (1) est une cartouche amovible interchangeable munie de moyens de fixation réversible avec la paroi (2).

8. Installation selon l'une quelconque des revendications précédentes, **caractérisée en ce que** la cartouche (1) est équipée de moyens de jonction réversible entre un support (3) et un substrat (4), ce dernier étant interchangeable.

## Claims

1. Heating, ventilation and/or air-conditioning unit comprising an air circuit (8) between at least one inlet vent and at least one outlet vent, this air circuit (8) being bounded by an air-impermeable volume bounded by a wall (2) that includes at least one opening (7) for the passage therethrough of a cartridge (1) for treating the air by diffusion of a volatile treating agent, a permeable partition (10) defining a chamber (11) for diffusion of the volatile agent being placed in the internal volume of the air circuit (8), the treating agent diffusing through the overall surface of the partition (10), **characterized in that** said partition is formed from a plastic filled with talc in a proportion of between 0 and 40% for a partition thickness of between 0.5 mm and 2 mm, said partition (10) is provided at least partly on the wall of the air circuit (8) and it does not matter whether said partition (10) is attached to and/or integrally molded with the wall (2) of the air circuit (8) in order to form a unitary part.

2. Unit according to Claim 1, **characterized in that**, when the opening (7) is provided through the wall (2) along an axis transverse to the direction of the air flow inside the circuit (8), the partition (10) lies in the extension of the opening (7) in the wall (2).

3. Unit according to Claim 1, **characterized in that**, when the opening (7) is provided through the wall (2) along an axis corresponding to the direction of air flow inside the circuit (8), the partition (10) lies transversely to the axis of the opening (7).

4. Unit according to any one of the preceding claims, **characterized in that** Lhe partition (10) is interposed between a substrate (4) of the cartridge (1) and the volume in which air flows through the circuit (8), the diffusion chamber (11) forming a housing for accommodating the substrate (4).

5. Unit according to any one of the preceding claims, **characterized in that** the cartridge (1) is fitted into the wall of a module (28) that houses at least air heat treatment means (30, 31).

6. Unit according to any one of the preceding claims, **characterized in that** the cartridge (1) is fitted into the wall of a duct (26, 35) that is located equally upstream and/or downstream of a module (28) that houses at least air heat treatment means (30, 31).

7. Unit according to any one of the preceding claims, **characterized in that** the cartridge (1) is an interchangeable removable cartridge provided with means for reversibly fastening it to the wall (2).

8. Unit according to any one of the preceding claims, **characterized in that** the cartridge (1) is equipped with reversible means for joining it between a support (3) and a substrate (4), the latter being interchangeable.

## Patentansprüche

1. Anlage zur Lüftung, Heizung und/oder Klimatisierung, umfassend einen Luftkreislauf (8) zwischen mindestens einer Eingangsmündung und mindestens einer Ausgangsmündung, wobei dieser Luftkreislauf (8) durch eine luftdichte Kapazität, die von einer Wand (2) umgeben ist, begrenzt ist, umfassend mindestens eine durch sie hindurchgehende Öffnung (7) für die Durchführung einer Kartusche (1) zur Luftbehandlung durch Diffusion eines flüchtigen Behandlungswirkstoffes, wobei eine durchlässige Trennwand (10), die eine Kammer (11) zur Diffusion des flüchtigen Wirkstoffes begrenzt, in dem Innenvolumen der Kapazität des Luftkreislaufes (8) angeordnet ist, wobei der Behandlungswirkstoff durch die Gesamtfläche der Trennwand (10) diffundiert, **dadurch gekennzeichnet, dass** die Trennwand von einem Kunststoff gebildet ist, der mit Talk in einem Verhältnis zwischen 0% und 40% bei einer Dicke der Trennwand zwischen 0,5 mm und 2 mm beaufschlagt ist, wobei die Trennwand (10) zumindest teilweise auf der Wand des Luftkreislaufes (8) vorgesehen ist, und dass die Trennwand (10) unterschiedslos auf die Wand (2) des Luftkreislaufes (8) aufgesetzt und/oder mit dieser mitgeformt ist, um ein einziges Stück zu bilden.

2. Anlage nach Anspruch 1, **dadurch gekennzeichnet, dass** die Öffnung (7) durch die Wand (2) entlang einer Querachse in Richtung der Luftzirkulation im Inneren des Kreislaufes (8) vorgesehen ist und sich die Trennwand (10) in der Verlängerung der Öffnung (7), die die Wand (2) umfasst, erstreckt.

3. Anlage nach Anspruch 1, **dadurch gekennzeichnet, dass** die Öffnung (7) durch die Wand (2) entlang einer Achse entsprechend der Luftzirkulation im Inneren des Kreislaufes (8) vorgesehen ist und sich die Trennwand (10) quer zur Achse der Öffnung (7) erstreckt.

4. Anlage nach einem der vorhergehenden Ansprüche, **dadurch gekennzeichnet, dass** die Trennwand (10) zwischen einem Substrat (4) der Kartusche (1) und dem Luftzirkulationsvolumen durch den Kreislauf (8) angeordnet ist, wobei die Diffusionskammer (11) eine Lagerung für die Aufnahme des Substrats (4) bildet.

5. Anlage nach einem der vorhergehenden Ansprüche, **dadurch gekennzeichnet, dass** die Kartusche (1) an der Wand eines Gehäuses (28) angebracht ist, das mindestens Mittel (30, 31) zur thermischen Behandlung der Luft enthält.

6. Anlage nach einem der vorhergehenden Ansprüche, **dadurch gekennzeichnet, dass** die Kartusche (1) an der Wand einer Leitung (26, 3b) angebracht ist, die sich unterschiedslos stromaufwärts und/oder stromabwärts zu einem Gehäuse (28) befindet, das mindestens Mittel (30, 31) zur thermischen Behandlung der Luft enthält.

7. Anlage nach einem der vorhergehenden Ansprüche, **dadurch gekennzeichnet, dass** die Kartusche (1) eine austauschbare abnehmbare Kartusche ist, die mit Mitteln zur reversiblen Befestigung an der Wand (2) versehen ist.

8. Anlage nach einem der vorhergehenden Ansprüche, **dadurch gekennzeichnet, dass** die Kartusche (1) mit Mitteln zur reversiblen Verbindung zwischen einem Träger (3) und einem Substrat (4) versehen ist, wobei dieses Letztgenannte austauschbar ist.
